# EUROPÄISCHE PATENTSCHRIFT

(11) **EP 2 841 031 B1**
(45) Veröffentlichungstag und Bekanntmachung des Hinweises auf die Patenterteilung: **13.07.2016**
(21) Anmeldenummer: 13721618.0
(22) Anmeldetag: 26.04.2013
(51) Int. Cl.: A61F 5/01

(54) **VERSCHLUSSELEMENT FÜR EINEN BAUCHGURT**
CLOSURE ELEMENT FOR ABDOMINAL BELT
ÉLÉMENT DE FERMETURE POUR UNE CEINTURE ABDOMINALE

(30) Priorität: 27.04.2012 DE 102012009250
(43) Veröffentlichungstag der Anmeldung: 04.03.2015
(73) Patentinhaber: Bauerfeind AG, 07937 Zeulenroda-Triebes (DE)
(72) Erfinder: HERTEL, Stefanie, 08527 Plauen (DE)
(74) Vertreter: Wohlfahrt, Jan Günther
(86) Internationale Anmeldenummer: PCT/EP2013/058716
(87) Internationale Veröffentlichungsnummer: WO 2013/160444

(56) Entgegenhaltungen:
- WO-A2-01/66051
- AU-B3- 622 098
- DE-A1-102005 031 867
- FR-A- 1 050 235
- US-A- 2 527 205
- US-A- 5 362 304
- US-A1- 2003 199 799

## Beschreibung

Die Erfindung betrifft ein flexibles Verschlusselement, zum Beispiel Bauchverschlusselement für Bandagen und Orthesen, sowie Verschlüsse und Produkte, beispielsweise Bauchverschlüsse von Bandagen und Orthesen, die solche Verschlusselemente aufweisen.

Verschlüsse aus zwei Verschlusselementen, beispielsweise Klettverschlüsse sind bekannt. Diese Verschlüsse können je nach Einsatzgebiet eine gewisse Breite aufweisen. Dies gilt beispielsweise für Bauchverschlüsse. Diese Breite kann die Verschlüsse in ungewünschter Weise unflexibel machen.

Bauchverschlüsse aus Bauchverschlusselementen für Bandagen und Orthesen sind beispielsweise aus der DE 10 2005 031 867 A1 bekannt. Diese werden häufig als Klettverschlüsse ausgestaltet. Die US 25 27 205 A und die US 2003/199799 A1 offenbaren darüber hinaus Bauchverschlusselemente mit Stabilisierungselementen, die plattenförmig sind und mit Deckschichten versehen sein können.

Solche Bauchverschlüsse müssen eine gewisse Breite haben um einen sicheren Halt zu gewähren. Auch sind sie für diesen sicheren Halt schwer biegbar. Bim Tragen der Orthese oder der Bandage insbesondere beim Vorbeugen des Oberkörpers oder beim Sitzen drückt der Bauchverschluss somit unangenehm in den Körper, insbesondere in den Bauch. So besteht bei bestehenden Bauchverschlüssen von Bandagen und Orthesen insbesondere der Nachteil, dass bei vielen Patienten der obere Rand der Verschlüsse in den Bauch oder auf die Rippenbögen und der untere Rand des Verschlusses in die Leistengegend drücken.

Die Erfindung hat sich die Aufgabe gestellt, Verschlüsse, insbesondere Bauchverschlüsse, bereitzustellen, die die Nachteile aus dem Stand der Technik überwinden, insbesondere die einen sicheren Halt beispielsweise einer Bandage oder Orthese ermöglichen und dennoch angenehm zu tragen sind, insbesondere bei Bewegungen und Positionen des Körpers, insbesondere Oberkörpers wie Vorbeugen, Sitzen oder Drehen.

Das der Erfindung zugrunde liegende technische Problem wird gelöst durch die Bereitstellung eines flexiblen Verschlusselements nach Anspruch 1.

Das der Erfindung zugrunde liegende technische Problem wird insbesondere gelöst durch die Bereitstellung eines flexiblen Verschlusselements, wobei das Verschlusselement zwei elastische Deckschichten aufweist, wobei sich zwischen den zwei elastischen Deckschichten ein plattenförmiges Stabilisierungselement befindet, wobei das plattenförmige Stabilisierungselement mindestens einen Schlitz aufweist, und wobei das plattenförmige Stabilisierungselement über mindestens der Hälfte der Länge des Stabilisierungselements an mindestens einer der beiden längsverlaufenden Kanten geschlitzt ist.

In einer bevorzugten Ausführungsform handelt es sich bei dem Verschlusselement um ein Bauchverschlusselement. Das Verschlusselement kann aber auch für andere Verschlüsse verwendet werden, insbesondere Verschlüsse die eine gewisse Breite aufweisen, beispielsweise ähnlich eines Bauchverschlusses. Bevorzugt handelt es sich um Verschlusselemente von Verschlüssen an medizinischen und orthopädischen Produkten oder Sportprodukten wie beispielsweise Orthesen oder Bandagen, insbesondere Rumpfbandagen, Rumpforthesen, Rückenbandagen, Rückenorthesen, Schulterbandagen, Schulterorthesen, Armbandagen, Armorthesen, Gelenksbandagen, Gelensorthesen, Kniebandagen oder Knieorthesen.

Beispielsweise eignen sich die erfindungsgemäßen Verschlusselemente nicht nur für Bauchverschlüsse an Bauchgurten, sondern auch an Verschlüssen von Schultergurten, insbesondere von breiten Schultergurten. Auch hier kann die durch die Schlitze erzeugte Flexibilität den Tragekomfort erhöhen, beispielsweise bei Schulterbewegungen und Drehbewegungen des Oberkörpers.

In einer bevorzugten Ausführungsform sind die obere Kante und die untere Kante des plattenförmigen Stabilisierungselements geschlitzt. In einer bevorzugten Ausführungsform bestehen die zwei elastischen Deckschichten aus einem textilen Material und das plattenförmige Stabilisierungselement aus einem Kunststoff. In einer bevorzugten Ausführungsform weist das plattenförmige Stabilisierungselement an mindestens einer der beiden längsverlaufenden Kanten Schlitze auf, die mindestens halb so breit sind wie die zwischen den Schlitzen gebildeten Stege. In einer bevorzugten Ausführungsform weist das plattenförmige Stabilisierungselement an mindestens einer der beiden längsverlaufenden Kanten Schlitze und zwischen den Schlitzen liegende Stege auf, wobei die Länge der Schlitze und der Stege mindestens ein zehntel der Höhe des Stabilisierungselements entspricht. In einer bevorzugten Ausführungsform ist das plattenförmige Stabilisierungselement mit den zwei elastischen Deckschichten verschweißt, verklebt oder vernäht. In einer bevorzugten Ausführungsform ist das Verschlusselement über eine Klettverbindung flexibel mit einem Gurt oder einer Bandage oder einer Orthese verbindbar. In einer bevorzugten Ausführungsform weist das Verschlusselement eine aufgesetzte Tasche auf.

Die Erfindung betrifft auch einen Verschluss, bestehend aus zwei erfindungsgemäßen Verschlusselementen. Bevorzugt ist der der Verschluss ein Bauchverschluss. Der Verschluss kann beispielsweise ein Klettverschluss, ein Hakenverschluss, ein Knopfverschluss oder ein Schnappverschluss sein.

Die Erfindung betrifft auch ein Produkt, beispielsweise einen Gurt, eine Bandage oder Orthese, enthaltend mindestens ein erfindungsgemäßes Verschlusselement oder einen erfindungsgemäßen Verschluss.

Ein erfindungsgemäßes flexibles Verschlusselement kann insbesondere ein Bauchverschlusselement sein. Die im Folgenden genannten alternativen und bevorzugten Ausführungsformen eines erfindungsgemäßen Bauchverschlusselements sind aber auch als alternative und bevorzugte Ausführungsformen eines erfindungsgemäßen flexiblen Verschlusselements zu verstehen, das kein Bauchverschlusselement ist.

Das der Erfindung zugrunde liegende technische Problem wird also insbesondere auch gelöst durch die Bereitstellung eines Bauchverschlusselements, wobei das Bauchverschlusselement mindestens zwei elastische Deckschichten aufweist, wobei sich zwischen den zwei elastischen Deckschichten ein plattenförmiges Stabilisierungselement befindet, wobei das plattenförmige Stabilisierungselement mindestens einen Schlitz aufweist.

In einer bevorzugten Ausführungsform weist das Bauchverschlusselement mindestens zwei elastische Deckschichten auf, wobei sich zwischen den zwei elastischen Deckschichten ein plattenförmiges Stabilisierungselement befindet, wobei das plattenförmige Stabilisierungselement über mindestens der Hälfte der Länge des Stabilisierungselements an mindestens einer der beiden längsverlaufenden Kanten geschlitzt ist.

Erfindungsgemäß weißt ein das plattenförmige Stabilisierungselement also mindestens einen Schlitz auf. Bevorzugt ist eine Vielzahl von Schlitzen.

Der mindestens eine Schlitz kann im Innenbereich des oder im Außenbereich des plattenförmigen Stabilisierungselements liegen. Der mindestens eine Schlitz kann also an einer Kante des plattenförmigen Stabilisierungselements enden. Er kann aber auch gänzlich von dem des plattenförmigen Stabilisierungselement umgeben sein. Bei mindestens zwei Schlitzen, insbesondere einer Vielzahl von Schlitzen können sich einige Schlitze an einer Kante des plattenförmigen Stabilisierungselements befinden und andere Schlitze gänzlich von dem des plattenförmigen Stabilisierungselement umgeben sein.

Die Schlitze können in jeglicher Richtung verlaufen, beispielsweise längs des plattenförmigen Stabilisierungselements oder quer zum plattenförmigen Stabilisierungselement. Die Schlitze können auch kreuzartig ausgeformt sein, beispielsweise durch zwei sich querende Schlitze. Die Schlitze können eine Länge von beispielsweise mindestens 1 cm und höchstens 20 cm haben Die Schlitze können eine Breite von beispielsweise mindestens 0 cm und höchstens 2 cm haben. Eine Breite von 0 cm wird erreicht, wenn ein Schlitz in das plattenförmige Stabilisierungselement nur eingeschnitten wird, aber kein Material aus dem plattenförmigen Stabilisierungselement ausgeschnitten wird.

In einer bevorzugten Ausführungsform ist das Bauchverschlusselement flexibel.

In einer bevorzugten Ausführungsform ist das Bauchverschlusselement für Bandagen und Orthesen geeignet.

Es zeigte sich überraschender weise, dass ein Aufbau eines Bauchverschlusselements aus zwei elastischen Deckschichten und einem zwischengelagerten plattenförmigen Stabilisierungselement, das geschlitzt ist, insbesondere das an mindestens einer der beiden längsverlaufenden Kanten geschlitzt ist, einen guten und sicheren Halt beispielsweise einer Bandage oder Orthese bietet und gut auftretende radiale Zugkräfte insbesondere durch das plattenförmigen Stabilisierungselement, das dem Bauchverschlusselement eine flächige Stabilität gibt, aufnimmt, aber durch die Schlitze in dem plattenförmigen Stabilisierungselement dennoch ein bequemes und angenehmes Tragen auch beim Beugen des Oberkörpers oder beim Sitzen ermöglicht wird. Durch die spezielle Konstruktion mit Schlitzen, bevorzugt im Randbereich des Bauchverschlusselements, wird eine komfortable elastische Zone gestaltet, die eine bessere Passform an die verschiedenen Körperformen ermöglicht. Weiterhin erlauben die elastischen Randzonen des Bauchverschlusselements eine uneingeschränkte Bauchatmung.

Es ist also bevorzugt vorgesehen, dass das plattenförmigen Stabilisierungselement am oberen und/oder am unteren Rand mit Schlitzen, mäanderförmigen oder nutenartigen Aussparungen versehen ist, die im Zusammenwirken mit den elastischen Deckschichten teilelastische Randbereiche mit den gewünschten und hier beschriebenen Eigenschaften ergeben.

In einer bevorzugten Ausführungsform sind die obere Kante und/oder die untere Kante des plattenförmigen Stabilisierungselements über mindestens die Hälfte geschlitzt. In einer bevorzugten Ausführungsform sind die obere Kante und/oder die untere Kante des plattenförmigen Stabilisierungselements über mindestens drei/viertel der Kantenlänge geschlitzt.

In einer bevorzugten Ausführungsform sind die obere Kante und die untere Kante des plattenförmigen Stabilisierungselements über mindestens die Hälfte geschlitzt. In einer bevorzugten Ausführungsform sind die obere Kante und die untere Kante des plattenförmigen Stabilisierungselements über mindestens drei/viertel der Kantenlänge geschlitzt.

In einer bevorzugten Ausführungsform sind die obere Kante und die untere Kante des plattenförmigen Stabilisierungselements geschlitzt.

In einer bevorzugten Ausführungsform bestehen die zwei elastischen Deckschichten aus einem textilen Material oder enthält dieses. Das textile Material kann insbesondere ein textiles Flächenmaterial sein, beispielsweise ein Velours, ein Gewebe, ein Gestrick oder ein Gewebe. In einer bevorzugten Ausführungsform besteht das plattenförmige Stabilisierungselement aus einem Kunststoff oder enthält dieses. Ein Fachmann kann ohne weiteres geeignete Kunststoffe in geeigneter Härte und Stärke wählen.

In einer bevorzugten Ausführungsform bestehen die zwei elastischen Deckschichten aus einem textilen Material und das plattenförmige Stabilisierungselement aus einem Kunststoff.

In einer bevorzugten Ausführungsform weist das plattenförmige Stabilisierungselement an mindestens einer der beiden längsverlaufenden Kanten Schlitze auf, die mindestens halb so breit sind wie die zwischen den Schlitzen gebildeten Stege. Bevorzugt kann die Breite der Schlitze mindestens 0,1 cm und höchsten 2 cm betragen, insbesondere etwa mindestens 0,2 cm und höchsten 1 cm.

In einer bevorzugten Ausführungsform weist das plattenförmige Stabilisierungselement an mindestens einer der beiden längsverlaufenden Kanten Schlitze und zwischen den Schlitzen liegende Stege auf, wobei die Länge der Schlitze und der Stege mindestens ein zehntel der Höhe des Stabilisierungselements entspricht. Bevorzugt kann die Länge der Schlitze mindestens 1 cm und höchsten 10 cm betragen, insbesondere etwa mindestens 2 cm und höchsten 6 cm.

In einer bevorzugten Ausführungsform ist das plattenförmige Stabilisierungselement mit den zwei elastischen Deckschichten verschweißt, verklebt oder vernäht. Das plattenförmige Stabilisierungselement kann aber auch einfach zwischen den zwei elastischen Deckschichten eingelegt sein, wob bevorzugt die zwei elastischen Deckschichten an ihren Kanten miteinander verschweißt, verklebt oder vernäht sind.

In einer bevorzugten Ausführungsform ist das Bauchverschlusselement über eine Klettverbindung flexibel mit einem Bauchgurt oder einer Bandage oder einer Orthese verbindbar. In einer bevorzugten Ausführungsform ist die Klettverbindung eine Klettmaulverbindung.

In einer bevorzugten Ausführungsform weist mindestens ein der beiden elastischen Deckschichten auf ihrer Außenseite eine Klettfläche auf.

In einer bevorzugten Ausführungsform weist das Bauchverschlusselement eine aufgesetzte Tasche auf. Diese eignet sich in bevorzugter Weise zum Eingreifen einer Hand, wodurch das Bauchverschlusselement gut ziehbar und mit einem zweiten Bauchverschlusselement verbindbar ist. Bevorzugt ist daher die Tasche eine der Handform angepasste Einschubtasche.

Die vorliegende Erfindung betrifft auch einen Bauchverschluss, bestehend aus mindestens einem erfindungsgemäßen Bauchverschlusselement, bevorzugt aus zwei erfindungsgemäßen Bauchverschlusselementen.

In einer bevorzugten Ausführungsform ist der Bauchverschluss ein Klettverschluss, ein Hakenverschluss, ein Knopfverschluss oder ein Schnappverschluss. Besonders bevorzugt ist der Bauchverschluss ein Klettverschluss.

Die vorliegende Erfindung betrifft auch eine Bandage oder Orthese, enthaltend mindestens ein erfindungsgemäßes Bauchverschlusselement, bevorzugt zwei erfindungsgemäße Bauchverschlusselemente, oder einen erfindungsgemäßen Bachverschluss.

Bevorzugt ist die Orthese eine Rumpforthese oder eine Rückenorthese.

Bevorzugte Ausführungsformen ergeben sich auch aus den Unteransprüchen.

Die Erfindung wird nachfolgend anhand des in den Zeichnungen dargestellten Ausführungsbeispiels näher erläutert, ohne dass eine Beschränkung der Erfindung erfolgt.

Dabei zeigen die Figuren:
Figur 1: Aufsicht auf ein plattenförmiges Stabilisierungselement mit Schlitzen eines erfindungsgemäßen Bauchverschlusselements;
Figur 2: Aufsicht auf die erste elastische Deckschicht eines erfindungsgemäßen Bauchverschlusselements;
Figur 3: Aufsicht auf die zweite elastische Deckschicht eines erfindungsgemäßen Bauchverschlusselements;
Figur 4: Aufsicht auf ein erfindungsgemäßes Bauchverschlusselement, bei dem eine der zwei elastischen Deckschichten teilweise aufgeklappt ist.

### Beispiel:

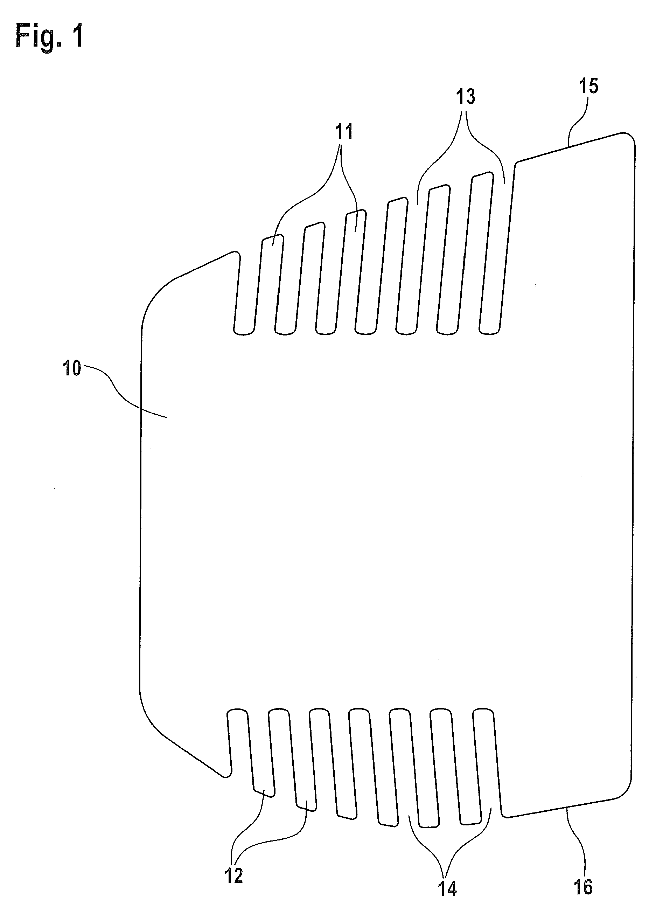

Figur 1 zeigt ein plattenförmiges Stabilisierungselement (10) mit Schlitzen oder Aussparungen (13) an der oberen Kante (15) des Stabilisierungselements (10) und mit Schlitzen oder Aussparungen (14) an der unteren Kante (16) des Stabilisierungselements (10). Zwischen den Schlitzen (13, 14) weist das Stabilisierungselement (10) Stege (11, 12) auf. Das Stabilisierungselement (10) ist einstückig und besteht aus einem Kunststoff.

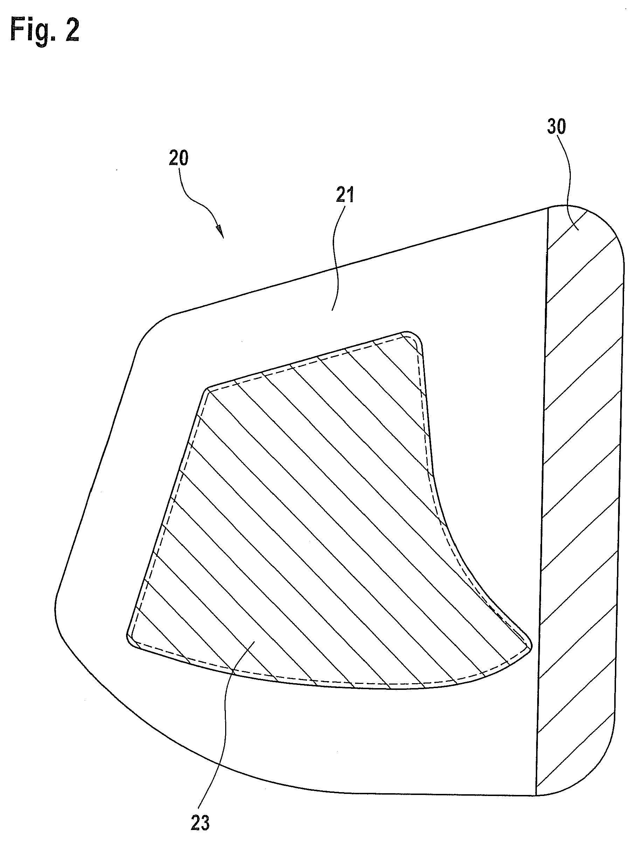

Figur 2 zeigt die Aufsicht auf die erste elastische Deckschicht (21) eines erfindungsgemäßen Bauchverschlusselements. Die erste Deckschicht (21) bildet mit einer zweiten Deckschicht, mit der sie zum Beispiel am Rand vernäht oder verklebt ist, eine Ummantelung oder Umhüllung (20) für ein plattenförmiges Stabilisierungselement. Auf die erste elastische Deckschicht (21) ist eine Tasche (23) aufgesetzt, beispielsweise aufgenäht oder aufgeklebt, die zum Eingreifen mit einer Hand geeignet ist. Ein Ende der Deckschicht (21) ist als Klettmaulverschluss (30) ausgestaltet, mit dem der Bauchverschluss an einen Gurt oder eine Orthese angebracht werden kann.

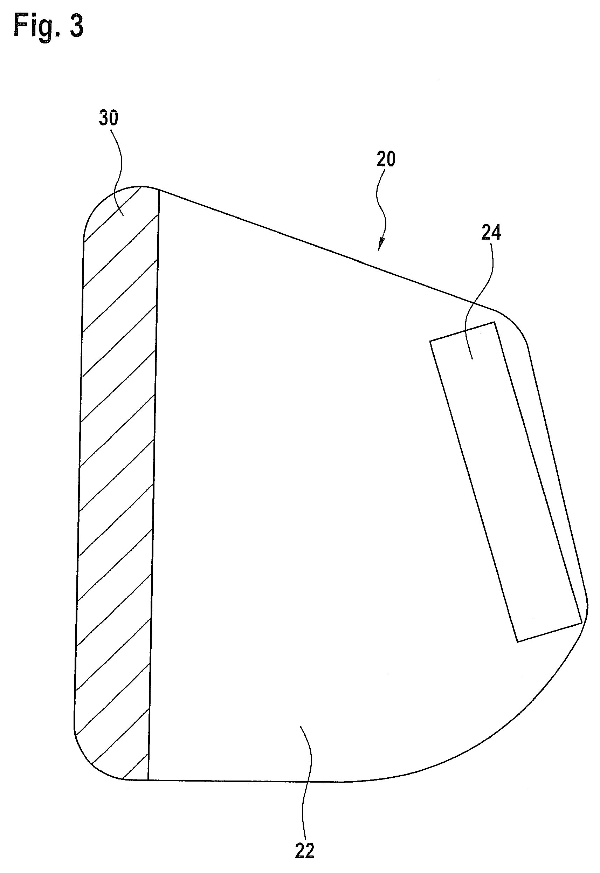

Figur 3 zeigt die Aufsicht auf die zweite elastische Deckschicht (22) eines erfindungsgemäßen Bauchverschlusselements. Die zweite Deckschicht (22) bildet mit der ersten Deckschicht aus Figur 2, mit der sie zum Beispiel am Rand vernäht oder verklebt ist, eine Ummantelung oder Umhüllung (20) für ein plattenförmiges Stabilisierungselement. Auf die zweite elastische Deckschicht (21) ist ein Klettverschluss (24) aufgesetzt, beispielsweise aufgenäht oder aufgeklebt, mit dem das Bauchverschlusselement mit einem zweiten Bauchverschlusselement, das ebenfalls einen Klettverschluss aufweist, zu einem Bauchverschluss verbunden werden kann. Ein Ende der Deckschicht (22) ist als Klettmaulverschluss (30) ausgestaltet, mit dem der Bauchverschluss an einen Gurt oder eine Orthese angebracht werden kann.

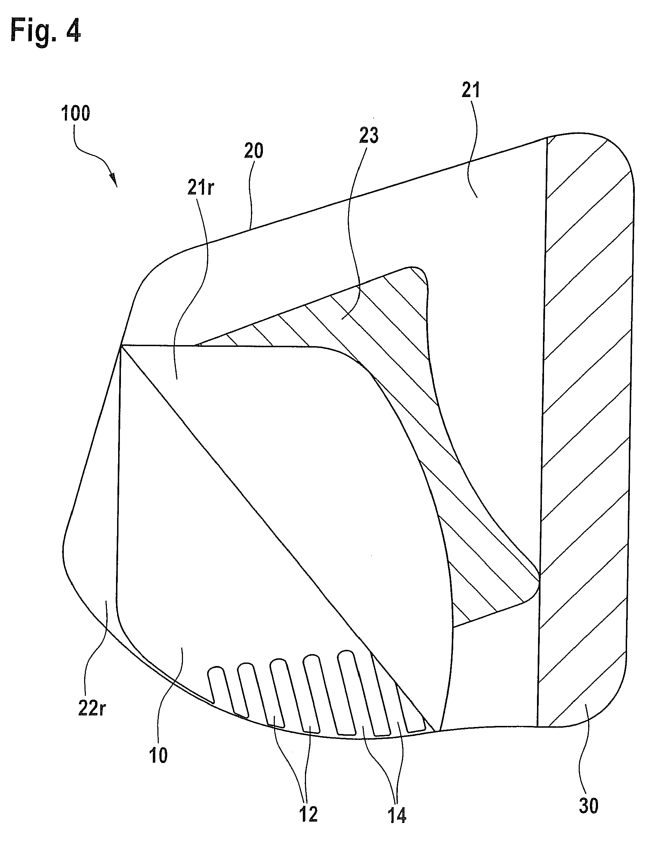

Figur 4 zeigt ein erfindungsgemäßes Bauchverschlusselement (100) mit der ersten elastischen Deckschicht (21) aus Figur 2, der zweiten elastischen Deckschicht (22) aus Figur 3 und dem plattenförmigen Stabilisierungselement (10) aus Figur 1. Die elastischen Deckschichten (21, 22) bestehen aus einem textilen Flächenmaterial.

Die erste elastische Deckschicht (21) ist teilweise aufgeklappt, um die Sicht auf das zwischen den beiden elastischen Deckschichten (21, 22) liegende plattenförmige Stabilisierungselement (10) zu ermöglichen. Damit sind auch die Innenseiten (21 r, 22r) der beiden Deckschichten zu sehen. Normalerweise sind das plattenförmige Stabilisierungselement (10) und die Innenseiten (21 r, 22r) nicht sichtbar, da die beiden elastischen Deckschichten (21, 22) zumindest an ihren Rändern umlaufend miteinander verbunden sind, beispielsweise verklebt oder vernäht sind.

Durch die spezielle Konstruktion mit Schlitzen (14) und Stegen (12) im unteren Randbereich und mit Schlitzen im oberen Randbereich (nicht sichtbar) des Stabilisierungselements (10) des Stabilisierungselements (10) wird eine komfortable elastische Zone gestaltet, die eine bessere Passform an die verschiedenen Körperformen ermöglicht. Weiterhin erlauben die so erhaltenen elastischen Randzonen des Bauchverschlusselements (100) eine uneingeschränkte Bauchatmung.

Die erste elastische Deckschicht (21) weist auf der Außenseite eine aufgesetzte der Handform angepasste Einschubtasche (23) auf. Diese eignet sich zum Eingreifen einer Hand, wodurch das Bauchverschlusselement beim Anziehen einer Bandage oder Orthese gut gezogen und mit einem zweiten Bauchverschlusselement verbunden werden kann.

An einem Ende des Bauchverschlusselements (100) befindet sich ein sich maulartig öffnendes Teilstück (30) mit innenliegenden Klettflächen. Dieses Teilstück (30) erlaubt es das Bauchverschlusselement (100) an einen Gurt, an einer Bandage oder an einer Orthese zu befestigen und bei Bedarf wieder zu lösen.

## Patentansprüche

1. Flexibles Verschlusselement (100) von einem Verschluss an einem medizinischen oder orthopädischen Produkt oder Sportprodukt, wobei das Verschlusselement (100) zwei elastische Deckschichten (21,22) aufweist, wobei sich zwischen den zwei elastischen Deckschichten (21,22) ein plattenförmiges Stabilisierungselement (10) befindet, **dadurch gekennzeichnet,**
**dass** das plattenförmige Stabilisierungselement (10) über mindestens der Hälfte der Länge des Stabilisierungselements (10) an mindestens einer der beiden längs verlaufenden Kanten (15,16) geschlitzt ist.

2. Verschlusselement nach Anspruch 1, wobei es sich um ein Bauchverschlusselement (100) handelt.

3. Verschlusselement nach einem der vorhergehenden Ansprüche, wobei die obere Kante (15) und die untere Kante (16) des plattenförmigen Stabilisierungselements (10) geschlitzt sind.

4. Verschlusselement nach einem der vorhergehenden Ansprüche, wobei die zwei elastischen Deckschichten (21,22) aus einem textilen Material und das plattenförmige Stabilisierungselement (10) aus einem Kunststoff bestehen.

5. Verschlusselement nach einem der vorhergehenden Ansprüche, wobei das plattenförmige Stabilisierungselement (10) an mindestens einer der beiden längsverlaufenden Kanten (15,16) Schlitze (13,14) aufweist, die mindestens halb so breit sind wie die zwischen den Schlitzen (13,14) gebildeten Stege (11,12).

6. Verschlusselement nach einem der vorhergehenden Ansprüche, wobei das plattenförmige Stabilisierungselement (10) an mindestens einer der beiden längsverlaufenden Kanten (15,16) Schlitze (13,14) und zwischen den Schlitzen (13,14) liegende Stege (11,12) aufweist, wobei die Länge der Schlitze (13,14) und der Stege (11,12) mindestens ein Zehntel der Höhe des Stabilisierungselements (10) entspricht.

7. Verschlusselement nach einem der vorhergehenden Ansprüche, wobei das plattenförmige Stabilisierungselement (10) mit den zwei elastischen Deckschichten (21,22) verschweißt, verklebt oder vernäht ist.

8. Bauchverschlusselement nach Anspruch 2, wobei das Bauchverschlusselement (100) über eine Klettverbindung (30) flexibel mit einem Bauchgurt oder einer Bandage oder einer Orthese verbindbar ist.

9. Verschlusselement nach einem der vorhergehenden Ansprüche, wobei das Verschlusselement (100) eine aufgesetzte Tasche (23) aufweist.

10. Verschluss, bestehend aus zwei Verschlusselementen nach einem der vorhergehenden Ansprüche.

11. Verschluss nach Anspruch 10, wobei der Verschluss ein Bauchverschluss ist.

12. Verschluss nach Anspruch 10 oder 11, wobei der Verschluss ein Klettverschluss, ein Hakenverschluss, ein Knopfverschluss oder ein Schnappverschluss ist.

13. Bandage oder Orthese, enthaltend mindestens einen Verschlusselement (100) nach einem der Ansprüche 1 bis 7 oder einen Verschluss nach einem der Ansprüche 10 bis 12.

## Claims

1. A flexible closure element (100) of a closure on a medical or orthopedic product or sports product, wherein the closure element (100) has two elastic cover layers (21, 22), wherein a sheet-type stabilizing element (10) is provided between the two elastic cover layers (21, 22), **characterized in that** the sheet-type stabilizing element (10) is slotted over at least half of the length of the stabilizing element (10) on at least one of the two edges running longitudinally (15, 16).

2. The closure element according to claim 1, wherein it is an abdominal closure element (100).

3. The closure element according to any one of the preceding claims, wherein the upper edge (15) and the lower edge (16) of the sheet-type stabilizing element (10) are both slotted.

4. The closure element according to any one of the preceding claims, wherein the two elastic cover layers (21, 22) are made of a textile material, and the sheet-type stabilizing element (10) is made of a plastic.

5. The closure element according to any one of the preceding claims, wherein the sheet-type stabilizing element (10) has slots (13, 14) on at least one of the two longitudinal edges (15, 16), these slots being at least one-half as wide as the webs (11, 12) formed between the slots (13, 14).

6. The closure element according to any one of the preceding claims, wherein the sheet-type stabilizing element (10) has slots (13, 14) on at least one of the two longitudinal edges (15, 16) and has webs (11, 12) provided between the slots (13, 14), wherein the length of the slots (13, 14) and the webs (11, 12) corresponds to at least one-tenth of the height of the stabilizing element (10).

7. The closure element according to any one of the preceding claims, wherein the sheet-type stabilizing element (10) is welded, glued or sewn to the two elastic cover layers (21, 22).

8. The abdominal closure element according to claim 2, wherein the abdominal closure element (100) can be connected flexibly to an abdominal belt or to a bandage or to an orthotic by means of a Velcro-type closure (30).

9. The closure element according to any one of the preceding claims, wherein the closure element (100) has an attached pocket (23).

10. The closure consisting of two closure elements according to any one of the preceding claims.

11. The closure according to claim 10, wherein the closure is an abdominal closure.

12. The closure according to claim 10 or 11, wherein the closure is a Velcro-type closure, a hook closure, a button closure or a snap closure.

13. The bandage or orthotic, comprising at least one closure element (100) according to any one of claims 1 to 7 or a closure according to any one of claims 10 to 12.

## Revendications

1. Élément de fermeture (100) flexible pour une fermeture sur un produit médical ou orthopédique ou un produit de sport, l'élément de fermeture (100) présentant deux couches de recouvrement (21, 22) élastiques, dans lequel entre les deux couches de recouvrement (21, 22) élastique se trouve un élément de stabilisation (10) sous forme de plaque, **caractérisé en ce que**
l'élément de stabilisation (10) sous forme de plaque est fendu sur au moins la moitié de la longueur de l'élément de stabilisation (10) au niveau d'au moins un des bords longitudinaux (15, 16).

2. Élément de fermeture selon la revendication 1, l'élément étant un élément de fermeture abdominale (100).

3. Élément de fermeture selon l'une quelconque des revendications précédentes, dans lequel le bord supérieur (15) et le bord inférieur (16) de l'élément de stabilisation (10) sous forme de plaque sont fendus.

4. Élément de fermeture selon l'une quelconque des revendications précédentes, dans lequel les couches de recouvrement (21, 22) élastiques consistent en matière textile, et l'élément de stabilisation (10) sous forme de plaque consiste en matière plastique.

5. Élément de fermeture selon l'une quelconque des revendications précédentes, dans lequel l'élément de stabilisation (10) sous forme de plaque présente des fentes (13, 14) sur au moins un des deux bords longitudinaux (15, 16), dont la largeur est au moins la moitié de la largeur des âmes (11, 12) formées entre les fentes (13, 14).

6. Élément de fermeture selon l'une quelconque des revendications précédentes, dans lequel l'élément de stabilisation (10) sous forme de plaque présente des fentes (13, 14) sur au moins un des deux bords longitudinaux (15, 16), et des âmes (11, 12) formées entre les fentes (13, 14), la longueur des fentes (13, 14) et des âmes (11, 12) étant au moins un dixième de la hauteur de l'élément de stabilisation (10).

7. Élément de fermeture selon l'une quelconque des revendications précédentes, dans lequel l'élément de stabilisation (10) sous forme de plaque est soudé, collé ou cousu avec les deux couches de recouvrement (21, 22) élastiques.

8. Élément de fermeture abdominale selon la revendication 2, dans lequel l'élément de fermeture abdominale (100) peut être raccordé de manière flexible avec une ceinture abdominale ou un bandage ou une orthèse au moyen d'une connexion de type velcro (30).

9. Élément de fermeture selon l'une quelconque des revendications précédentes, dans lequel l'élément de fermeture (100) présente une poche appliquée (23).

10. Fermeture consistant en deux éléments de fermeture selon l'une des revendications précédentes.

11. Fermeture selon la revendication 10, la fermeture étant une fermeture abdominale.

12. Fermeture selon la revendication 10 ou 11, la fermeture étant une fermeture de type velcro, une fermeture à crochets, une fermeture à boutons ou une fermeture encliquetable.

13. Bandage ou orthèse, contenant au moins un élément de fermeture (100) selon l'une quelconque des revendications 1 à 7 ou une fermeture selon l'une quelconque des revendications 10 à 12.
